# EUROPEAN PATENT APPLICATION

(11) **EP 4 528 279 A1**
(43) Date of publication of application: **26.03.2025**
(21) Application number: 23382969.6
(22) Date of filing: 25.09.2023
(51) Int. Cl.: G01N 33/68

(54) **IN VITRO METHOD FOR THE DIAGNOSIS, PROGNOSIS AND TREATMENT RESPONSE OF MIGRAINE**

(71) Applicant: Fundación Instituto de Investigación Sanitaria de Santiago de Compostela, 15706 Santiago de Compostela, A Coruña (ES); Servizo Galego de Saude, 15703 Santiago de Compostela (ES)
(72) Inventor: OURO VILLASANTE, Alberto, 15706 Santiago de Compostela (ES); SOBRINO MOREIRAS, Tomás, 15706 Santiago de Compostela (ES); LEIRA MUÍÑO, Rogelio, 15706 Santiago de Compostela (ES); LEIRA FEIJÓO, Yago, 15706 Santiago de Compostela (ES); CASTILLO SÁNCHEZ, Jose, 15706 Santiago de Compostela (ES)
(74) Representative: Hoffmann Eitle

(57) **Abstract**

The present invention refers to the medical field. Particularly, the present invention refers to *in vitro* methods based on the expression of Autotaxin (ATX) for the diagnosis and/or prognosis of migraine, for the differential diagnosis between patients suffering from chronic migraine and patients suffering from episodic migraine, for the prognosis of pain intensity or pain duration in patients suffering from migraine, and for assessing whether a patient suffering from migraine will respond to treatment with botulinum toxin.

## Description

### FIELD OF THE INVENTION

The present invention refers to the medical field. Particularly, the present invention refers to *in vitro* methods based on the level of expression of autotaxin (ATX), or of the gene ENPP2, for the diagnosis and/or prognosis of migraine, for the differential diagnosis between patients suffering from chronic migraine and patients suffering from episodic migraine, for the prognosis of pain intensity or pain duration in patients suffering from migraine, and/or for assessing whether a patient suffering from migraine will respond to a treatment, preferably a preventive treatment such as botulinum toxin.

### STATE OF THE ART

Migraine is a type of headache characterized by recurrent attacks of moderate to severe throbbing and pulsating pain on one side of the head. It is the most common neurological disorder and the second most disabling human condition, whose pathogenesis is favored by a combination of genetic, epigenetic, and environmental factors.

In current clinical practice, migraine is diagnosed according to the International Headache Society (International Classification of Headache Disorders, ICHD-3), which recognizes two types of migraine according to their frequency: episodic migraine and chronic migraine. Chronic migraine is defined as a headache occurring on 15 or more days per month for more than 3 months that has the features of migraine headache on at least 8 days per month. Although the evolution of this classification system reflects an increasing understanding of the heterogeneity and variable clinical features of migraine, the diagnosis and treatment remain inadequate.

The lack of reliable biomarkers constitutes one of the main barriers to the precision diagnosis and treatment of migraine. In recent years, several efforts have been made to identify reliable biomarker(s) useful to monitor disease activity and/or ascertain the response to a specific treatment. Several circulating biomarkers have been proposed as diagnostic or therapeutic tools in migraine, mostly related to migraine's inflammatory pathophysiological aspects. Nonetheless, their detection is still a challenge for the scientific community, reflecting, at least in part, disease complexity and clinical diagnostic limitations.

So, there is an unmet medical need of identifying biomarkers for the diagnosis, prognosis, and prediction of treatment response of migraine. The present invention is aimed at solving this problem, and a biomarker that enables the diagnosis, prognosis, and prediction of response to treatment with botulinum toxin is herein provided.

### DESCRIPTION OF THE INVENTION

### Brief description of the invention

The present invention refers to *in vitro* methods, based on the level of expression of ATX, or of the gene ENPP2, in a biological sample obtained from a subject (preferably blood, serum or plasma), for the diagnosis and/or prognosis of migraine. So far, the diagnosis between episodic and chronic migraine is based on clinical data. However, it is estimated that 2.5% of patients with episodic migraines develop chronic migraines, a process commonly known as migraine transformation. Several studies have been focused on identifying factors and /or biomarkers associated with this process. Our invention has found significantly different ATX values between chronic and episodic migraine patients, as well as much higher than in healthy patients. In this sense, ATX can be used to determine between patients suffering from chronic migraine and patients suffering from episodic migraine, for the prognosis of pain intensity or pain duration in patients suffering from migraine, and/or for assessing whether a patient suffering from migraine will respond to treatment with botulinum toxin, preferably a preventive treatment such as botulinum toxin. In addition, to determine the possibility that a patient with episodic migraine will evolve into chronic migraine.

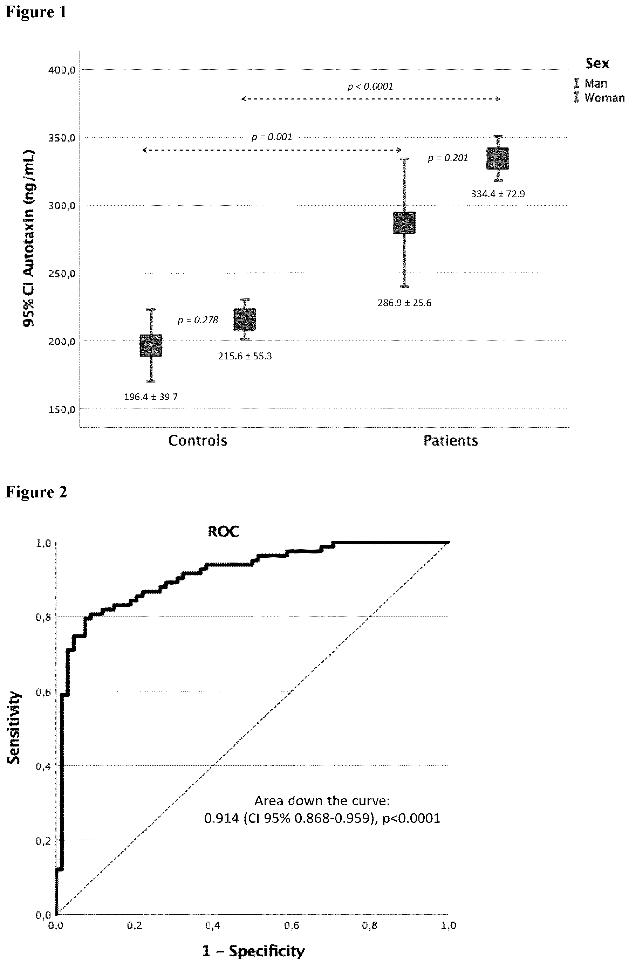

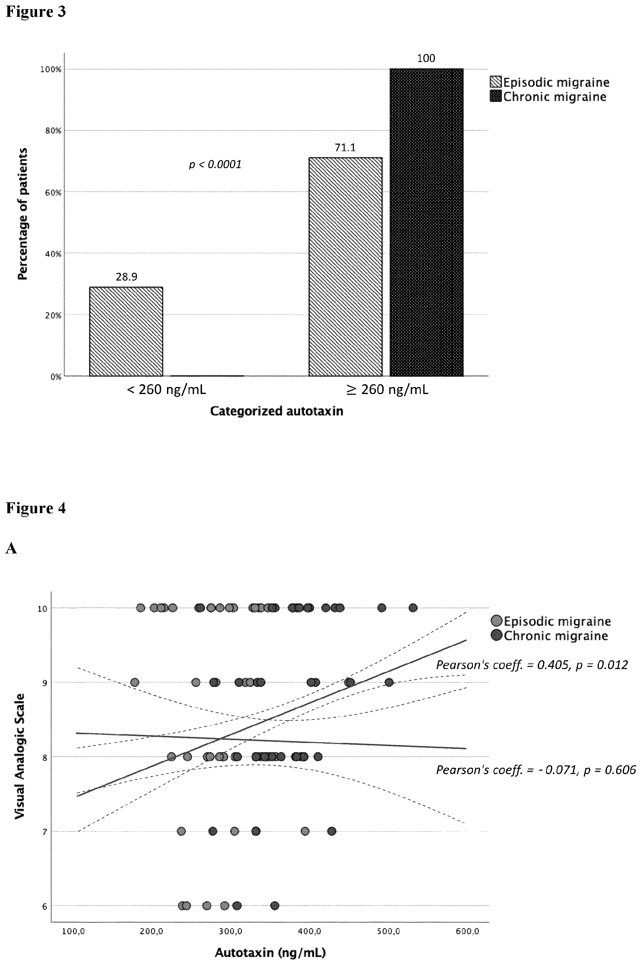

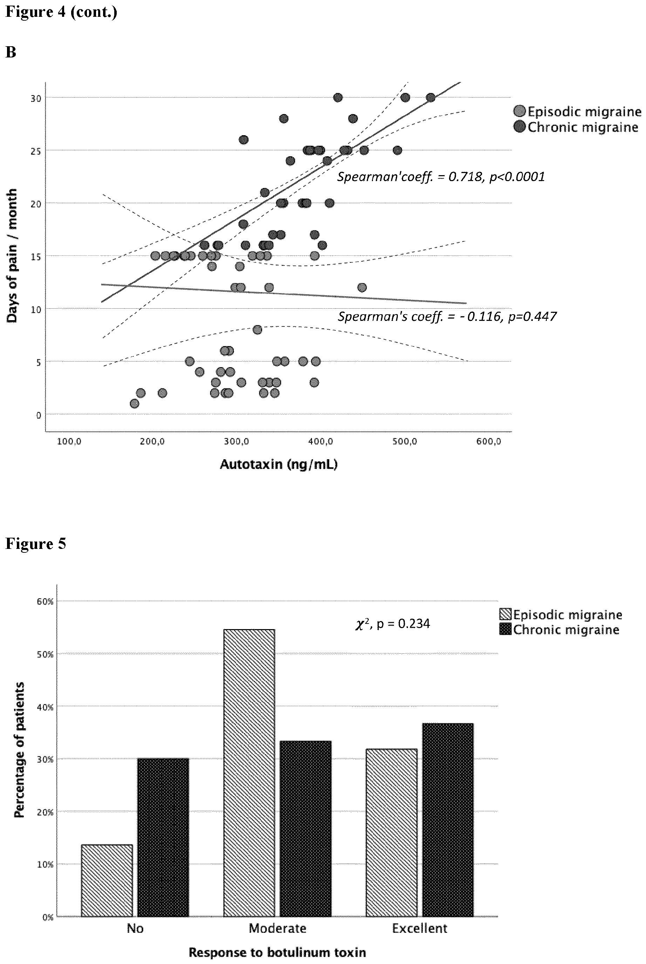

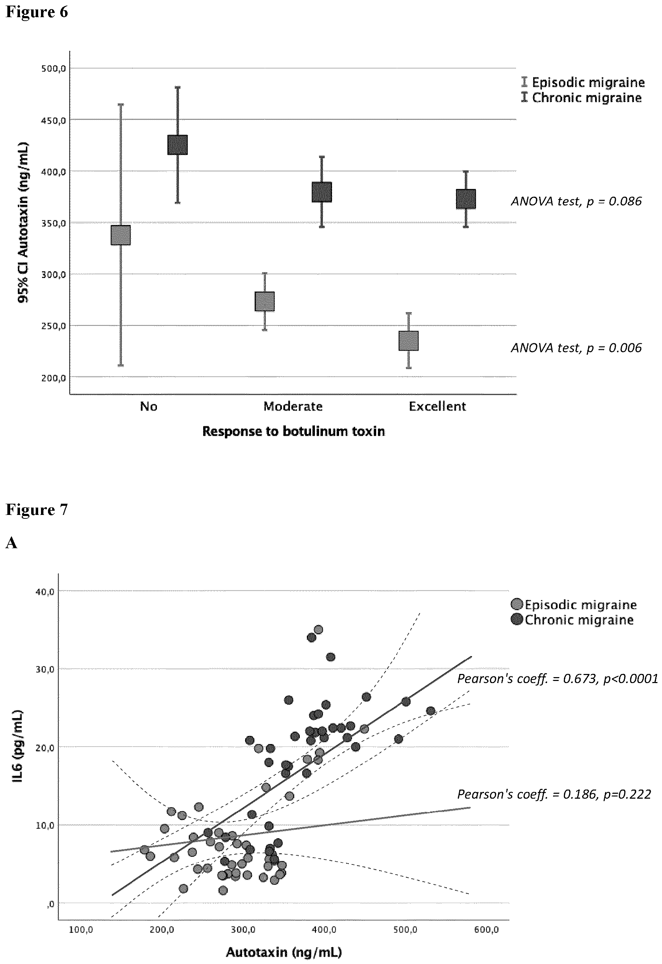

By quantifying the concentration levels of ATX in healthy subjects, as well as in individuals suffering from episodic and chronic migraines, the inventors of the present invention identified that:
- Serum ATX levels are significantly increased in patients suffering from migraine compared to controls (**Figure 1**).
- Serum ATX levels greater than 260 ng/mL enable the identification of patients suffering from migraine with a sensitivity of 85% and a specificity of 81% (**Figure 2**).
- Serum ATX levels are significantly increased in patients with chronic migraine compared to episodic migraine (332.1 ± 72.1 vs. 212.5 ± 53.3 ng/mL, p < 0.0001), indicating that serum ATX levels can be used to discriminate between episodic and chronic migraine.
- Pain intensity (**Figure 4A**) and days of pain (**Figure 4B**) are associated with higher ATX serum concentrations in patients with chronic migraine.
- ATX serum concentrations are higher in patients with a poor response to preventive treatment with botulinum toxin, especially in patients suffering from episodic migraines (**Figure 6**).

The first embodiment of the present invention refers to an *in vitro* method for the diagnosis and/or prognosis of migraine, which comprises: a) assessing the expression level of the protein ATX, or of the gene ENPP2, in a biological sample obtained from a subject, and b) wherein an increased expression level, as compared with the pre-established threshold value, is an indication that the subject is suffering from migraine or that the subject has a poor prognosis.

The second embodiment of the present invention refers to an *in vitro* method for the differential diagnosis between patients suffering from chronic migraine and patients suffering from episodic migraine that can evolve into chronic migraine, which comprises: a) assessing the expression level of the protein ATX, or of the gene ENPP2, in a biological sample obtained from the patients, and b) wherein an increased expression level, as compared with a pre-established threshold value, is an indication that the patients are suffering from chronic migraine.

The third embodiment of the present invention refers to an *in vitro* method for the prognosis of pain intensity or pain duration in patients suffering from migraine, which comprises: a) assessing the expression level of the protein ATX, or of the gene ENPP2, in a biological sample obtained from the patients, and b) wherein an increased expression level, as compared with the pre-established threshold value, is an indication that the patients will experience more intense and durable pain.

The fourth embodiment refers to an *in vitro* method for assessing whether a patient suffering from migraine will respond to treatment with botulinum toxin and/or for recommending treatment with botulinum toxin, which comprises: a) assessing the expression level of the protein ATX, or of the gene ENPP2, in a biological sample obtained from the patient, and b) wherein an increased expression level, as compared with the pre-established threshold value, is an indication that the patient will not respond to the treatment and/or that treatment with botulinum toxin is not recommended; or b) wherein a reduced expression level, as compared with the pre-established threshold value, is an indication that the patient will respond to the treatment and/or that treatment with botulinum toxin is recommended.

In a preferred embodiment, the migraine is chronic or episodic migraine.

In a preferred embodiment, the biological sample is selected from the group comprising: plasma, serum, or blood.

The fifth embodiment refers to botulinum toxin for use in the treatment of migraine, wherein the treatment comprises assessing whether a patient suffering from migraine will respond to treatment by following the method described above.

In a preferred embodiment, the botulinum toxin is for use in the preventive treatment of migraine.

In a preferred embodiment, the migraine is episodic migraine.

The sixth embodiment refers to the *in vitro* use of ATX, of the gene ENPP2, or of a kit comprising reagents to detect them, for the diagnosis of subjects suffering from migraine, for the differential diagnosis between patients suffering from chronic migraine and patients suffering from episodic migraine, for the prognosis of subjects suffering from episodic migraine that can evolve into chronic migraine, for assessing whether a patient suffering from migraine will respond to treatment with botulinum toxin and/or for recommending treatment with botulinum toxin.

Alternatively, the present invention refers to a method for treating migraine which comprises the administration of a therapeutically effective dose or amount of botulin toxin, wherein the treatment comprises assessing whether a patient suffering from migraine will respond to a treatment by following the method described above.

On the other hand, the present invention also refers to a method for detecting the level of expression of ATX in a test sample from a human subject at risk of developing migraine, the method comprising: a) contacting the biological sample with a reagent capable of specifically binding ATX; and b) measuring the level of ATX in the biological sample.

In a preferred embodiment, the present invention is a computer-implemented invention, wherein a processing unit (hardware) and a software are configured to: a) receive level of expression values of ATX or of the gene ENPP2, b) process the level of expression values in search of substantial variations or deviations, and c) provide an output through a terminal display of the variation or deviation of the level of expression values, wherein the variation or deviation of the level of expression values indicates that the subject may be suffering from migraine.

Finally, the present invention also refers to a computer program or a computer-readable media containing means for carrying out a method as herein defined.

### In the context of the present invention the following terms are defined:

- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- By "therapeutically effective dose or amount" of the pharmaceutical composition of the invention is intended an amount that, when administered as described herein, brings about a positive therapeutic response in a subject having cancer. The exact amount required will vary from subject to subject, depending on the age, and general condition of the subject, the severity of the condition being treated, the mode of administration, and the like. An appropriate "effective" amount in any individual case may be determined by one of ordinary skill in the art using routine experimentation, based upon the information provided herein.
- According to the present invention, the "pre-established threshold" value refers to a value previously determined:
   ∘ For the diagnosis and/or prognosis of migraine: in subjects who do not suffer from chronic migraine nor episodic migraine.
   ∘ For the differential diagnosis of patients suffering from chronic migraine from patients suffering from episodic migraine: by comparing subjects who suffer from chronic migraine with subjects that suffer from episodic migraine.
   ∘ For the differential diagnosis of patients suffering from episodic migraine from patients suffering from episodic migraine that can evolve into chronic migraine: by comparing subjects who suffer from chronic migraine with subjects that suffer from episodic migraine that evolved or not into chronic migraine.
   ∘ For the prognosis of pain intensity or pain duration in patients suffering from migraine: in patients who suffer from chronic migraine and episodic migraine.
   ∘ For assessing whether a patient suffering from migraine will respond to a treatment with botulinum toxin and/or for recommending a treatment with botulinum toxin: in patients who suffer from chronic or episodic migraine.
The "pre-established threshold value" can be determined experimentally, empirically or theoretically. Thus, for instance, the subject is likely to suffer from migraine or from chronic migraine when a higher level of expression of ATX is identified, as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Description of the figures

**Figure 1****.** Representation of ATX serum levels in controls and migraine patients discriminated by gender.
**Figure 2****.** Representation of receiver operating characteristic curve (ROC curve) to analyze the sensibility and specificity of ATX serum levels.
**Figure 3****.** Patients with chronic migraine (CM) and episodic migraine (EM) based on levels greater or less than 260 ng/mL of ATX.
**Figure 4****. A.** Correlation between the visual analogic scale and ATX serum levels in patients with chronic (CM) and episodic (EM) migraine. Determining the Pearson coefficient. **B.** Correlation between days of pain per month and ATX levels in patients with CM and EM. Determination of the Pearson coefficient.
**Figure 5****.** Representation of the percentage of patients based on their response to treatment with botulinum toxin.
**Figure 6****.** Correlation between the degree of response to botulinum toxin treatment and ATX levels in CM and EM patients. Determining the Pearson coefficient.

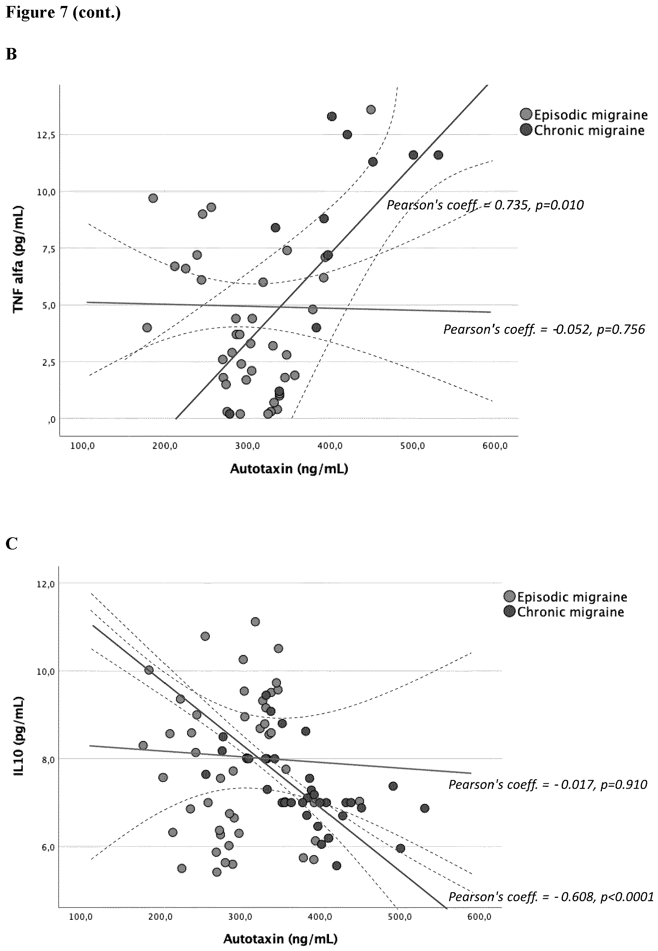

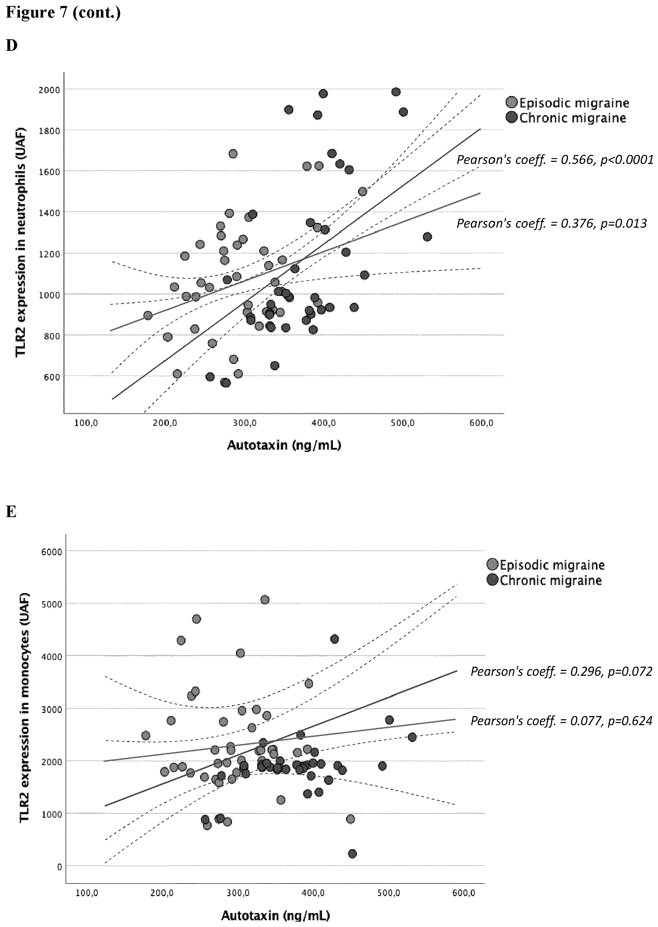

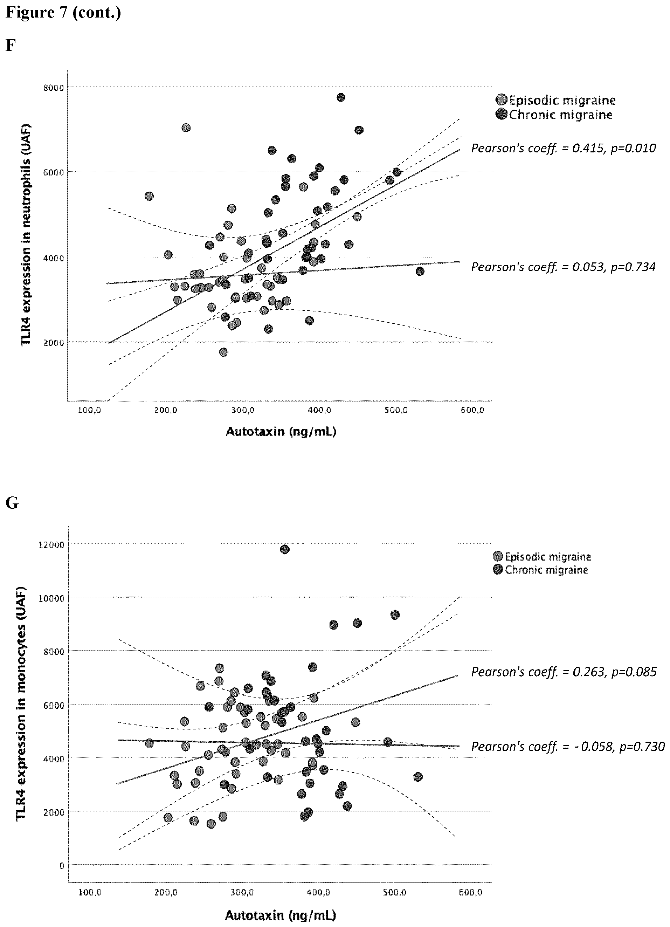

**Figure 7****. A.** Correlation between serum IL6 levels and ATX levels in CM patients and EM in plasma. **B.** Correlation between serum TNFα levels and ATX levels in CM patients and EM in plasma. **C.** Correlation between serum IL10 levels and ATX levels in CM patients and EM in plasma. **D.** Correlation between the expression of TLR2 in neutrophils and the serum levels of ATX in CM patients and EM. **E.** Correlation between the expression of TLR2 in monocytes and the levels of ATX in CM patients and EM in plasma. **F.** Correlation between the expression of TLR4 in neutrophils and the levels of ATX in CM patients and EM in plasma. **G.** Correlation between the expression of TLR4 in monocytes and the levels of ATX in CM patients and EM in plasma. In the statistical study, the Pearson coefficient was analyzed to determine the correlation between the variables.
**Figure 8****.** Correlation between S100beta serum levels and ATX levels in CM patients and EM in plasma. Determining the Pearson coefficient.
**Figure 9****.** Correlation between CGRP serum levels and ATX levels in CM patients and EM in plasma. Determining the Pearson coefficient.
**Figure 10****.** Correlation between PTX-3 serum levels and ATX levels in CM patients and EM in plasma. Determining the Pearson coefficient.
**Figure 11****. A.** Correlation between cellular fibronectin serum levels and ATX levels in CM patients and EM in plasma. **B.** Correlation between sTWEAK levels and ATX levels in CM patients and EM in plasma. Determining the Pearson coefficient.
**Figure 12****.** Schematic representation of the two proposed theories on the biological model of the relationship between ATX and migraine episodes.

### Detailed description of the invention

The present invention is illustrated by means of the Examples set below without the intention of limiting its scope of protection.

### Example 1. Material and Methods

### Example 1.1. Subjects

All patients were recruited prospectively from the outpatient Headache clinic of our Neurology Department and at *Hospital Clinico Universitario of Santiago de Compostela.* In total, 69 controls and 83 cases matched by age (p=0.233), but not by sex (p=0.022). 45 patients diagnosed with episodic and 38 with chronic migraine according to the International Classification of Headache Disorders, 3rd edition criteria, were selected.

### Example 1.2. Study protocol

Patients with episodic and chronic migraine, and healthy control subjects, all ≧18 years old who signed the informed consent, were included. Molecular markers were determined in peripheral venous blood. Migraineurs were headache-free from 72 hours prior the visit to 24 hours after the blood sample collection. If a migraine occurred within the first 24 hours, measurements were repeated in another headache-free period. Subjects had not previously consumed anti-inflammatory or analgesic medication.

Exclusion criteria were the following: 1) chronic inflammatory conditions; 2) severe systemic diseases; 3) pregnancy or lactation; 4) neuroendocrine tumors; 6) multisystemic trauma; 6) other neurological diseases; 7) vascular risk factors (arterial hypertension, diabetes mellitus, coronary disease, smoking, dyslipidemia and obesity with BMI ≧ 35 kg/m2); 8) excessive sport activity and 9) other forms of chronic headache.

### Example 1.3. Clinical variables

All subjects fulfilled a complete medical record including demographic data (age, gender), personal and family history, physical examination and neuroimaging. For migraineurs, type of migraine (with or with our aura), time of evolution of the disease (determined in years), intensity of headaches (measured by the visual analogic scale [VAS]), duration of attacks (quantified in hours) and frequency of headaches (number of days with pain per month) were recorded. These clinical parameters were considered as an average of the patient's episodes.

### Example 1.4. Laboratory tests

Markers of brain damage (S 100B), inflammation (interleukin-6, [IL-6], tumor necrosis factor alpha, [TNF-α], and intereleukin-10 [IL-10]), TGV activation (calcitonin gene-related peptide, [CGRP]), endothelial dysfunction (pentraxin-3, [PTX-3]) and BBB disruption (fibronectin, [cFn] and sTWEAK) as well as ATX were investigated.

For serum levels determination, 4.5 ml of blood from the antecubital vein was collected. Samples were obtained during a pain-free interval and after an overnight fast. All samples were kept in chemical test tubes and centrifuged at 3000 rpm for 15 minutes. Serum was immediately frozen and stored at -80°C for further analysis.

S 100B serum levels were determined using commercial ELISA kits (Labor Diagnostika Nord GmbH & Co. KG, Nordhom, Germany). Serum levels of IL-6, IL-10, and TNF-a were measured by using an immunodiagnostic IMMULITE 1000 System (Siemens Healthcare Global, Los Angeles, CA, USA). Serum levels of PTX3 (Assay Biotech, Sunnyvale, CA, USA), cFn (Cusabio Life Science, Wuhan, China). CGRP (Phoenix Pharmaceuticals Inc., Burlingame, CA, USA) and ATX (R&D Systems) were determined with commercial ELISA kits following manufacturer instructions. sTWEAK was measured using a Human TWEAK Elisa kit (Elabscience, Texas, USA). All the ELISA reactions were measured with a Biotek HT plate reader. Biomarkers were evaluated within the first 3 months after blood sample collection. The intra-assay and inter-assay coefficients of variation (CV) for all molecular markers were <8%.

On the other hand, blood samples collected in EDTA-anticoagulated tubes during interictal periods were extracted to determine the expression of TLR2 and TLR4 on neutrophils (TLR2N, TLR4N) and monocytes (TLR2M, TLR4M) by flow cytometry (FACSAria IIu flow cytometer (BD Biosciences, New Jersey, USA)). For the expression analysis of TLR2 and TLR4, monocytes, lymphocytes and neutrophils were separated by their forward and side scattering signal characteristics. Fluorescein isothiocyanate (FITC) anti-TLR2-conjugated monoclonal antibodies and phycoerythrin (PE) anti-TLR4 conjugated monoclonal antibodies were used to quantify TLR expression. Isotype-matched antibody controls were used to detect nonspecific staining. Red blood cells were lysed with BD lysing solution (BD Biosciences, New Jersey, USA). The expression of TLR2 and TLR4 in monocytes and neutrophils was analyzed using the FACS Diva 6.02 software (BD Biosciences, New Jersey, USA). Results were expressed as arbitrary fluorescence units (AFU).

All molecular and cellular determinations were performed in a laboratory blinded to clinical data.

### Example 1.5. Endpoints

The primary endpoint was the comparison of serum ATX levels between migraine patients and healthy control subjects, as well as between chronic and episodic migraineurs.

As secondary endpoints we analyzed the association of ATX serum levels with type of migraine (with or with our aura), time of evolution of the disease (determined in years), intensity of headaches (measured by the visual analogic scale [VAS]), duration of attacks (quantified in hours) and frequency of headaches (number of days with pain per month).

The association of ATX serum levels with the efficacy of preventive treatments, such as Onabotulinumtoxin A, was recorded as a drug response biomarker.

Finally, we analyzed the correlation between ATX and serum levels of endogenous ligands and inflammation biomarkers in order to investigate the possible mechanism of action of ATX in migraines.

### Example 1.6. Data and statistical analysis

Results were expressed as percentages for categorical variables and mean and standard deviation or median and percentiles 25 and 75 for continuous variables, depending on the normal or non-normal distribution of data. Normality was determined by Kolmogorov-Smirnov test. Statistical tests used to compare continuous data were independent t-test, Mann-Whitney U test, Kruskal-Wallis H, or ANOVA test. Categorical variables were reported as percentages and compared by X2test. Pearson coefficient for continuous variables (duration of disease) and Spearman coefficient for categorical variables (intensity of pain and frequency of attacks) were applied. Logistic regression analysis was used for determining significance and independence of the molecular markers. A value of p <0.05 was considered significant. Statistical analysis was performed with SPSS 27.0 for Mac software.

### Example 1.7. Ethics

The Ethics Committee of the Hospital Clinico Universitario of Santiago de Compostela (Spain) approved the protocol (2016/085). All patients and healthy controls were appropriately informed and signed consent.

### Example 2. Results

### Example 2.1. Descriptive study

69 controls and 83 patients, matched by age (41.2 ± 12.5 vs. 43.6 ± 12.1 years, p=0.233), but not by sex (controls: 11 men and 58 women; patients: 4 men and 79 women, p=0.022).

49 patients with migraine without aura, 27 with aura and 14 with mixed forms. Of them, 45 had episodic migraine and 38 chronic migraines.

Characteristics of migraine:
- Time of evolution from the beginning of migraines: 20.0 ± 14.3 months.
- Pain intensity (VAS): 9 [8 - 10].
- Frequency of crisis (days with pain per month): 14.3 ± 8.4 days.
- Average duration of migraine attacks: 16.8 ± 25.4 hours.

45 patients received prophylactic treatment with Onabotulinumtoxin A, 17 with an excellent response (more than 75% reduction in crisis), 20 with a moderate response (50-75% reduction in crisis) and 8 with no response.

### Example 2.2. Primary endpoints

### Comparison of serum ATX levels between migraine patients and control subjects.

ATX concentrations were significantly higher in migraine patients than in controls (332.1 ± 72.1 vs. 212.5 ± 53.3 ng/mL, p < 0.0001). Although the values were higher in women than in men, the statistical difference between controls and patients remained even after stratifying by sex (**Figure 1**).

ATX levels greater than 260 ng/mL identified patients with migraine versus controls with a sensitivity of 85% and a specificity of 81% (**Figure 2**).

In a logistic regression model, using case/control as the dependent variable, the sex-adjusted ATX ≥ 260 ng/mL presents an OR of 19.70 (CI 95% 8.45 - 45.94), p < 0.0001 (**Table 1**).

**Table 1: Dependent variable: case/control**

| | OR Not adjusted | CI 95% | p | OR Adjusted | CI 95% | p |
|---|---|---|---|---|---|---|
| ATX ≥ 260 ng/mL | 20.77 | 9.02 - 47.83 | <0.0001 | 19.70 | 8.45 - 45.94 | <0.0001 |
| Woman | 3.75 | 1.14 - 12.36 | 0.030 | 1.56 | 0.36 - 6.81 | 0.553 |

This means that ATX levels greater than 260 ng/mL (adjusted by sex) multiply by 20 the possibility that the sample corresponds to a patient and not to a control.

### Comparison of serum ATX levels between episodic and chronic migraine patients

ATX levels were significantly higher in patients with chronic migraine than with episodic migraine (375.7 ± 60.4 vs. 295.2 ± 59.7 ng/mL, p<0.0001). All patients with chronic migraine had ATX levels ≥ 260 ng/mL, compared to 71.1% of patients with episodic migraine (p<0.0001, **Figure 3**).

Age (p = 0.303), sex (p = 0.375) and time since the onset of the disease (p = 0.102) were similar in patients with episodic and chronic migraine.

### Example 2.3. Secondary endpoints

### Association between ATX levels and the clinical characteristic of migraine

Neither in patients with episodic or chronic migraine, were ATX levels associated with the type of migraine (without area, with aura or mixed) (p=0.544 vs. 0.265), with the time since the onset of the disease (p=0.503 vs. 0.500), nor with the duration of the crises (p=0.735 vs. 0.575).

However, in patients with chronic migraine, the relationship of ATX levels with pain intensity (**Figure 4A**) and with the number of days of pain (**Figure 4B**) was highly significant.

### Association between ATX levels and the efficacy of treatment with botulinum toxin

The response to botulinum toxin was worse, although not statistically significant, in patients with chronic migraines compared to those with episodic migraines (**Figure 5**).

ATX levels were higher in patients with poor response to treatment with botulinum toxin (p_{episodic}=0.006 , p_{chronic}=0.086, **Figure 6**).

### Correlation between ATX and serum levels of endogenous ligands and inflammation biomarkers

To investigate a possible mechanism of action of ATX in migraines, the inventors assessed the correlation between ATX levels and serum levels of endogenous ligands and inflammation biomarkers.

ATX levels correlated with serum levels of II,6 and TNF alpha (positive correlation), and with the levels of IL10 (negative correlation) in patients with chronic migraine, while no significant correlation between ATX and those inflammation markers was detected in patients with episodic migraine (Figures 7A-C).

In a logistic regression model to assess the cause/effect relationship between ATX and inflammatory biomarkers in chronic migraine, the inclusion of IL6 nullified the significance of ATX (**Table 2**), so it is possible to hypothesize that ATX is responsible for the increase of the proinflammatory cytokines and that these conditions directly influence the chronicity of migraine.

**Table 2: Dependent variable: episodic vs. chronic migraine**

| | OR Not adjusted | CI 95% | p | OR Adjusted | CI 95% | p |
|---|---|---|---|---|---|---|
| ATX | 1.02 | 1.01 - 1.03 | <0.0001 | 1.01 | 0.98 - 1.03 | 0.501 |
| IL6 | 1.20 | 1.11-1.29 | <0.0001 | 1.28 | 1.00 - 1.64 | 0.048 |
| TNFα | 1.34 | 1.09 - 1.64 | 0.005 | 0.88 | 0.61 - 1.25 | 0.467 |
| IL 10 | 0.76 | 0.54 - 1.06 | 0.759 | | | |

They also investigated the correlation between ATX levels and TLR2 or between ATX and TLR4 expression, in neutrophils and monocytes (Figures 7E-G). An either significant or almost significant correlation was detected between ATX and TLR2 (in neutrophils and monocytes), and between ATX and TLR4 (in neutrophils, but not in monocytes) in patients with chronic migraine. In patients with episodic migraine, a significant correlation was detected for ATX and TLR2 in neutrophils (**Figure 7D**), and for ATX and TLR4 expression in monocytes (**Figure 7G**).

Therefore, immune marker expression in neutrophils is related to ATX in chronic migraine, but its association is not demonstrated in logistic regression models (**Table 3**).

**Table 3: Dependent variable: episodic vs. chronic migraine**

| | OR Not adjusted | CI 95% | p | OR Adjusted | CI 95% | p |
|---|---|---|---|---|---|---|
| ATX | 1.02 | 1.01 - 1.03 | <0.0001 | 1.03 | 1.01 - 1.05 | <0.0001 |
| TLR2N | 1.00 | 0.99 - 1.00 | 0.358 | | | |
| TLR2M | 0.99 | 0.99 - 1.01 | 0.058 | | | |
| TLR4N | 1.00 | 0.97 - 1.00 | 0.116 | | | |
| TLR4M | 1.00 | 0.99 - 1.00 | 0.122 | | | |

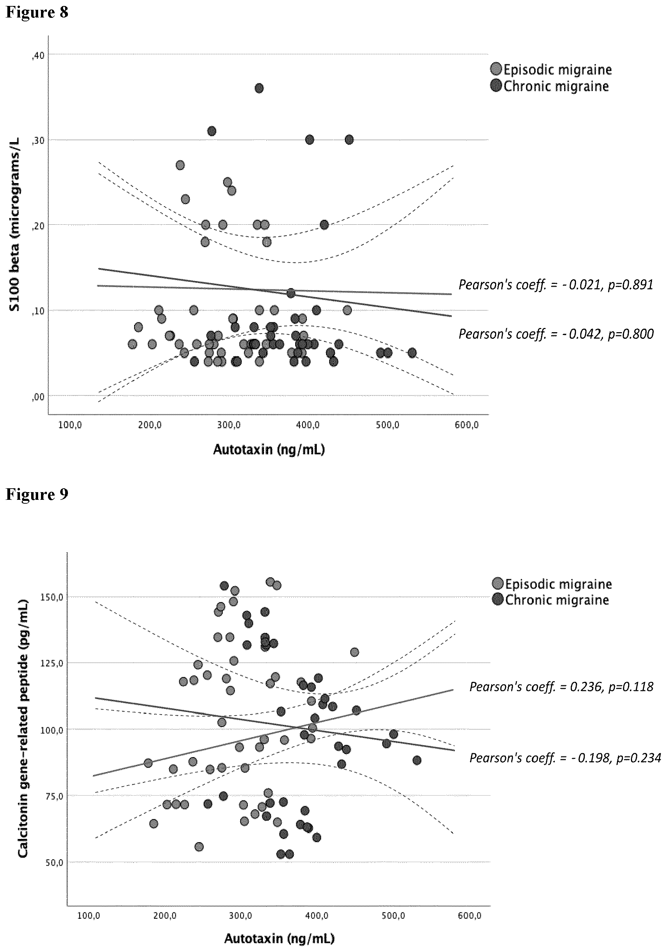

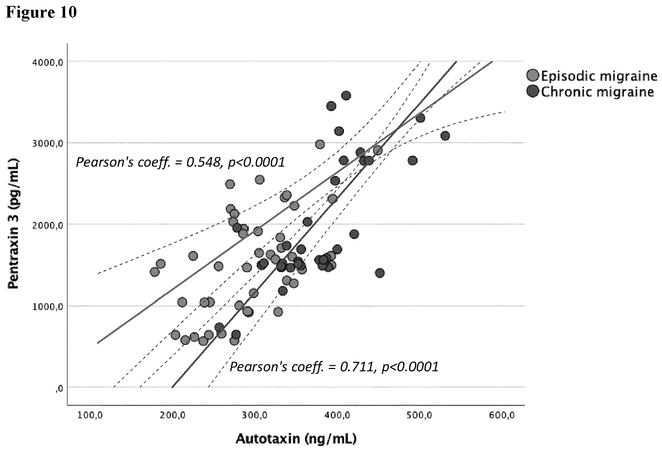

No correlation was detected between ATX levels and plasma S100 beta (brain damage marker, **Figure 8**) or calcitonin gene-related peptide (trigemino-vascular system activation marker, **Figure 9**) in patients with episodic or chronic migraine, while a significant correlation was detected between ATX and pentraxin 3 (endothelial dysfunction marker, **Figure 10**). Therefore, brain injury biomarkers and trigemino-vascular system activation were not associated with ATX levels.

**Table 4: Dependent variable: episodic vs. chronic migraine**

| | \| OR | CI 95% | p | OR | CI 95% | p |
|---|---|---|---|---|---|---|
| | Not adjusted | | | Adjusted | | |
| ATX | 1.02 | 1.01 - 1.03 | <0.0001 | 1.03 | 1.02 - 1.04 | <0.0001 |
| Pentraxin 3 | 1.01 | 1.00 - 1.02 | 0.014 | 0.99 | 0.98 - 1.00 | 0.176 |

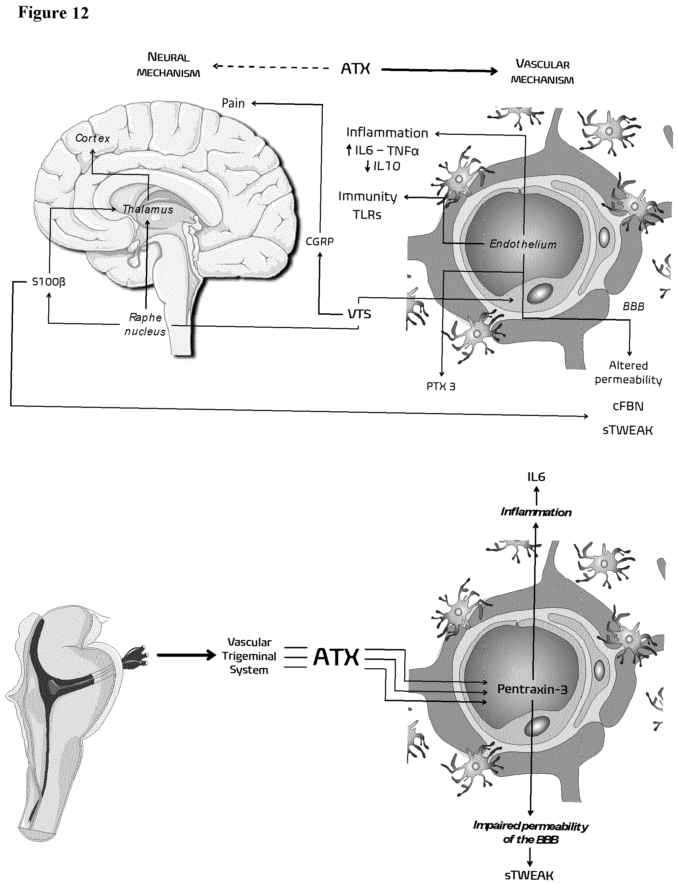

The significant association between the levels of pentraxin 3 and ATX, both in patients with episodic and chronic migraine, enables the inventors to suggest a hypothesis that is expressed in the last figure (**Figure 12**).

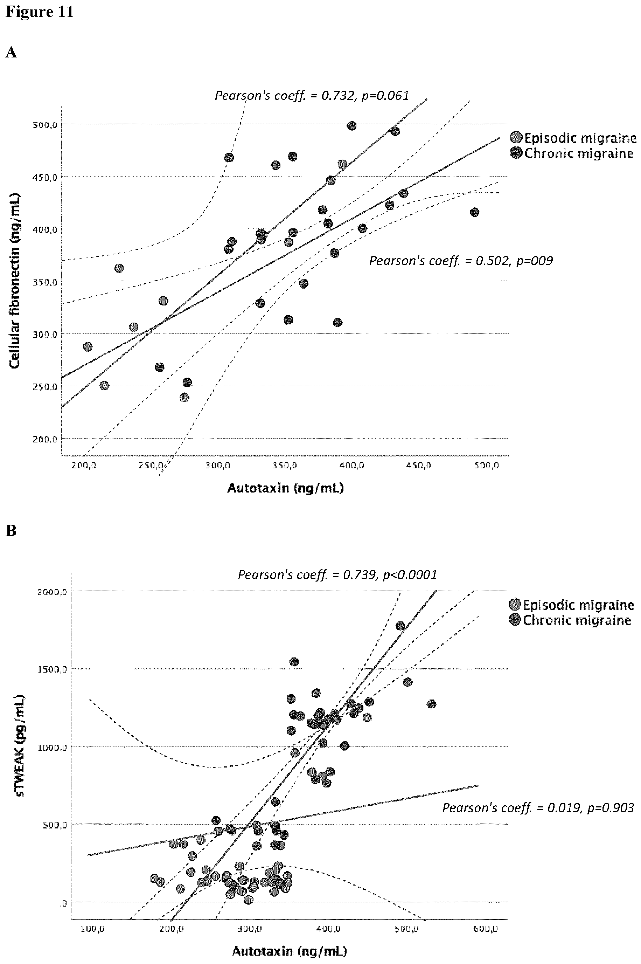

Finally, a significant correlation was detected between ATX and cellular fibronectin in episodic and chronic migraine, and between ATX and sTWEAK in episodic, but not in chronic migraine (Figures 11A-B). Both cellular fibronectin and sTWEAK play a role in the blood-brain barrier.

**Table 5: Dependent variable: episodic vs. chronic migraine**

| | OR Not adjusted | CI 95% | p | OR Adjusted | CI 95% | p |
|---|---|---|---|---|---|---|
| ATX | 1.02 | 1.01 - 1.03 | <0.0001 | 1.01 | 0.99 - 1.02 | 0.328 |
| sTWEAK | 1.01 | 1.00 - 1.02 | <0.0001 | 1.01 | 1.00 - 1.02 | 0.004 |

## Claims

1. *In vitro* method for the diagnosis and/or prognosis of migraine, which comprises:
a) Assessing the expression level of the protein Autotaxin, or of the gene ENPP2, in a biological sample obtained from a subject, and
b) Wherein an increased expression level, as compared with a pre-established threshold value, is an indication that the subject is suffering from migraine or that the subject has a poor prognosis.

2. *In vitro* method for the differential diagnosis between patients suffering from chronic migraine and patients suffering from episodic migraine, which comprises:
a) Assessing the expression level of the protein Autotaxin, or of the gene ENPP2, in a biological sample obtained from the patients, and
b) Wherein an increased expression level, as compared with a pre-established threshold value, is an indication that the patients are suffering from chronic migraine.

3. *In vitro* method for the prognosis of pain intensity or pain duration in patients suffering from migraine, which comprises:
a) Assessing the expression level of the protein Autotaxin, or of the gene ENPP2, in a biological sample obtained from the patients, and
b) Wherein an increased expression level, as compared with a pre-established threshold value, is an indication that the patients will experience more intense and durable pain.

4. *In vitro* method for assessing whether a patient suffering from migraine will respond to treatment with botulinum toxin and/or for recommending treatment with botulinum toxin, which comprises:
a) Assessing the expression level of the protein Autotaxin, or of the gene ENPP2, in a biological sample obtained from the patient, and
b) Wherein an increased expression level, as compared with a pre-established threshold value, is an indication that the patient will not respond to the treatment and/or that treatment with botulinum toxin is not recommended; or b) wherein a reduced expression level, as compared with the pre-established threshold value, is an indication that the patient will respond to the treatment and/or that treatment with botulinum toxin is recommended.

5. *In vitro* method, according to any of the claims 1, 3 or 4, wherein the migraine is chronic or episodic migraine.

6. *In vitro* method, according to any of the previous claims, wherein the biological sample is selected from the group comprising: plasma, serum or blood.

7. Botulinum toxin for use in the treatment of migraine, wherein the treatment comprises assessing whether a patient suffering from migraine will respond to treatment by following the method of claims 4 or 5.

8. Botulinum toxin for use, according to claim 7, in the preventive treatment of migraine.

9. Botulinum toxin for use, according to claims 7 or 8, wherein the migraine is episodic migraine.

10. *In vitro* use of Autotaxin, of the gene ENPP2, or of a kit comprising reagents to detect them, for the diagnosis of subjects suffering from migraine, for the differential diagnosis between patients suffering from chronic migraine and patients suffering from episodic migraine, for the prognosis of subjects suffering from migraine, for assessing whether a patient suffering from migraine will respond to treatment with botulinum toxin and/or for recommending treatment with botulinum toxin.
